# FASCICULE DE BREVET EUROPEEN

(11) **EP 2 947 328 B1**
(45) Date de publication et mention de la délivrance du brevet: **05.12.2018**
(21) Numéro de dépôt: 15305543.9
(22) Date de dépôt: 13.04.2015
(51) Int. Cl.: F04D 25/06, A61M 16/00, F04D 29/08, F04D 29/62, H02K 7/14, F04D 25/08, F04D 29/42, H02K 5/22

(54) **MICRO-SOUFFLANTE À ÉTANCHÉITÉ AMÉLIORÉE POUR VENTILATEUR MÉDICAL ET SON PROCÉDÉ D'ASSEMBLAGE**
MIKROGEBLÄSE MIT VERBESSERTER DICHTIGKEIT FÜR MEDIZINISCHEN VENTILATOR, UND ZUGEHÖRIGES ZUSAMMENBAUVERFAHREN
MICROFAN WITH IMPROVED SEAL FOR MEDICAL VENTILATOR AND METHOD FOR ASSEMBLING THE SAME

(30) Priorité: 21.05.2014 FR 1454585
(43) Date de publication de la demande: 25.11.2015
(73) Titulaire: Air Liquide Medical Systems, 92160 Antony (FR)
(72) Inventeur: Dutheil, Benjamin, 75001 Paris (FR); Dubois, Pierre-Emmanuel, 92330 Sceaux (FR); Fishman, Clara, 94800 Villejuif (FR); Gadrey, Sébastien, 58230 Gouloux (FR); Guiducci, Hadrien, 75018 Paris (FR)
(74) Mandataire: Pittis, Olivier

(56) Documents cités:
- EP-A2- 2 000 675
- WO-A1-2011/017763
- FR-A- 1 399 239
- FR-A1- 2 908 482

## Description

L'invention porte sur une micro-soufflante destinée à être utilisée dans un appareil médical d'assistance respiratoire, dont l'étanchéité fluidique a été améliorée, ainsi que sur son procédé d'assemblage et sur un appareil médical d'assistance respiratoire incorporant une telle micro-soufflante.

Dans un appareil médical d'assistance respiratoire, encore appelé ventilateur médical, il est usuel d'utiliser un micro-compresseur, aussi appelé « micro-soufflante », ou encore « compresseur » ou « turbine » par abus de langage, qui permet d'aspirer de l'air et de le distribuer ensuite au patient auquel est raccordé le ventilateur. Ceci se fait via une connectique adaptée comprenant classiquement un ou des tuyaux flexibles et une interface patient, tel un masque respiratoire ou analogue.

Une micro-soufflante ou compresseur permet en fait de générer un flux d'air, c'est-à-dire un débit d'air, éventuellement enrichi en oxygène, à une pression adéquate qui sont nécessaires à une bonne ventilation d'un patient donné.

Habituellement, une micro-soufflante comprend un moteur électrique avec un arbre rotatif portant une roue à ailettes. Le moteur est protégé extérieurement par un capotage ou carter externe. La roue à ailette est prise en sandwich entre deux demi-coques, appelées volutes inférieure et supérieure, qui forment entre elles un compartiment renfermant la roue à ailette. La volute supérieure est généralement surmontée d'une âme acoustique et d'un pavillon, ou peut intégrer ces éléments. La volute inférieure peut, dans certains modes de réalisation, venir ceinturer le capotage ou du carter du moteur.

Des exemples de micro-soufflantes sont décrits par exemple par les documents EP-A-2102504, EP-A-2165078, EP-A-2506907 et EP-A-2122180.

De même, le document FR-A-2908482 enseigne une micro-soufflante avec moteur électrique entraînant une roue à ailette agencée dans un compartiment entre des volutes inférieure et supérieure, ledit compartiment surmontant le carter du moteur.

Habituellement, l'assemblage d'une micro-soufflante prend beaucoup de temps et est fastidieux car beaucoup d'opérations manuelles sont nécessaires et certaines sont très longues, voire délicates.

Plus précisément, l'assemblage d'une micro-soufflante commence généralement par un montage de la roue à ailettes sur l'arbre du moteur à l'aide d'une machine spécifique, cette pose se faisant avec un certain jeu en hauteur entre roue et carter du moteur.

Ensuite, les volutes inférieure et supérieure sont agencées autour de la roue à ailettes en positionnant très précisément la volute supérieure par rapport à la roue. En effet, un positionnement précis est indispensable car il est directement lié aux bonnes performances de la micro-soufflante. Ce problème est encore plus notable lorsque l'on utilise une roue non flasquée pour gagner en inertie et en légèreté. Les volutes prennent donc la roue à ailette « en sandwich », telles deux demi-coques.

Puis, afin d'obtenir une étanchéité fluidique de l'ensemble, il est primordial de combler non seulement l'espace entre les bords des deux volutes mais aussi celui entre la volute inférieure et le capotage ou carter externe du moteur. Pour ce faire, l'espace entre les deux volutes est généralement comblé par collage, alors que celui entre la volute inférieure et le moteur par apposition d'un joint entre ces éléments. Cette étape d'étanchéité fluidique est plutôt longue et fastidieuse, et doit être opérée avec minutie car tout défaut de comblement conduit à un défaut d'étanchéité.

Ainsi, en pratique, il a été constaté, dans certaines micro-soufflantes, des défauts d'étanchéité entre les bords des volutes résultait souvent d'un mauvais alignement entre eux, ou entre la volute inférieure et le capotage externe du moteur du fait d'une compression insuffisante ou imparfaite du joint d'étanchéité résultant notamment de l'architecture actuelle de ces pièces dans les micro-soufflantes existantes.

Le problème qui se pose est de proposer une structure de micro-soufflante améliorée qui permette d'améliorer l'étanchéité fluidique entre la volute inférieure et le capotage externe du moteur et de préférence aussi entre les deux volutes, mais sans compliquer le montage global de la micro-soufflante, voire même en le simplifiant, ainsi qu'un procédé d'assemblage d'une telle micro-soufflante, et un appareil d'assistance respiratoire comprenant une telle micro-soufflante.

La solution de la présente invention est alors une micro-soufflante, c'est-à-dire un compresseur d'air, pour appareil médical de ventilation assistée, comprenant un moteur électrique à arbre rotatif portant une roue à ailettes, ledit moteur électrique étant compris dans un capotage périphérique, ladite roue à ailettes étant agencée entre une volute inférieure et une volute supérieure, la volute inférieure comprenant un passage central venant ceinturer la surface périphérique du capotage, caractérisée en ce que :
- le capotage du moteur comprend un rebord périphérique supérieur se projetant radialement en éloignement par rapport à la surface périphérique externe du capotage,
- le passage central de la volute inférieure est bordé intérieurement par un épaulement annulaire, un joint d'étanchéité annulaire étant agencé sur ledit épaulement annulaire,
- la volute inférieure comprend en outre plusieurs butées internes portées par la surface interne de la volute inférieure,
- le rebord périphérique supérieur du capotage du moteur étant positionné entre le joint d'étanchéité annulaire et les butées internes de la volute inférieure, lesdites butées internes venant appuyer sur ledit rebord périphérique supérieur du capotage du moteur de manière à obtenir une compression du joint d'étanchéité annulaire entre ledit rebord périphérique supérieur du capotage et l'épaulement annulaire de la volute inférieure, et
- le rebord périphérique supérieur du capotage du moteur comprend plusieurs évidements dimensionnés pour permettre le passage des butées internes de la volute inférieure.

Selon le cas, la micro-soufflante de l'invention peut comprendre l'une ou plusieurs des caractéristiques techniques suivantes :
- le nombre d'évidements est supérieur ou égal au nombre de butées.
- les butées internes de la volute inférieure se projettent radialement en éloignement par rapport à la surface interne de la volute inférieure.
- la volute inférieure comprend au moins 3 butées internes.
- la volute inférieure comprend en outre une bordure périphérique supérieure comprenant plusieurs logements, le bord périphérique inférieur de la volute supérieure comprenant plusieurs picots, chaque picot venant se loger dans l'un des logements lorsque les volutes inférieure et supérieure sont assemblées l'une à l'autre.
- la bordure périphérique supérieure de la volute inférieure comprenant au moins 3 logements et le bord périphérique inférieur de la volute supérieure comprenant au moins 3 picots, de préférence le nombre de logements est égal au nombre de picots.
- la volute supérieure est surmontée d'une âme acoustique et/ou d'un pavillon.

L'invention concerne en outre un appareil d'assistance respiratoire comprenant une micro-soufflante selon l'invention, ainsi que l'utilisation d'un tel appareil d'assistance respiratoire pour administrer un gaz respiratoire, en particulier de l'air ou de l'air enrichi en oxygène, à un patient souffrant d'une insuffisance ou d'un trouble respiratoire.

Par ailleurs, l'invention porte aussi sur un procédé d'assemblage d'une micro-soufflante selon l'invention, caractérisé en ce que l'on procède selon les étapes successives suivantes :
a) on agence la volute inférieure munie du joint d'étanchéité autour du capotage du moteur en insérant le capotage du moteur dans le passage central de la volute inférieure,
b) on positionne chaque butée portée par la volute inférieure en vis-à-vis d'un évidement correspondant porté par le rebord périphérique supérieur du capotage,
c) on opère un mouvement de translation de la volute inférieure en direction du rebord périphérique supérieur du capotage pour faire passer les butées au travers desdits évidements correspondants, et
d) après qu'à l'étape c), les butées aient complètement traversé les évidements, on opère un mouvement de rotation de la volute inférieure par rapport au capotage du moteur de manière à décaler angulairement les butées par rapport aux évidements et à positionner chaque butée en regard d'une partie du rebord périphérique supérieur du capotage de manière à ce que le rebord périphérique supérieur du capotage soit pris en sandwich entre lesdites butées et le joint d'étanchéité porté par l'épaulement annulaire de la volute inférieure, en assurant ainsi un maintien solidaire et fluidiquement étanche du capotage du moteur à la volute inférieure.

Selon le cas, le procédé de l'invention peut comprendre l'une ou plusieurs des caractéristiques techniques suivantes :
- préalablement à l'étape a), on positionne la roue à ailettes sur l'arbre rotatif du moteur.
- après l'étape d), il comprend l'étape e) d'assembler les volutes inférieure et supérieure, l'une à l'autre, en insérant les picots portés par le bord périphérique inférieur de la volute supérieure au sein des logements correspondants portés par la bordure périphérique supérieure de la volute inférieure.
- après l'étape e), il comprend l'étape f) de fixer une âme acoustique et/ou un pavillon à la volute supérieure.
- après le positionnement la roue à ailettes sur l'arbre rotatif du moteur, on opère un équilibrage de ladite roue à ailettes sur ledit arbre rotatif.

L'invention va maintenant être mieux comprise grâce à la description détaillée suivante, faite à titre illustratif mais non limitatif, en référence aux figures annexées parmi lesquelles :
- la Figure 1 représente une vue schématique d'une micro-soufflante pour ventilateur médical selon l'invention,
- les Figure 2a et 2b sont des vues latérales de la micro-soufflante de la Figure 1 débarrassée de l'âme acoustique et du pavillon, montrant la volute supérieure solidarisée (Fig. 2a) ou désolidarisée (Fig. 2b) de la volute inférieure,
- la Figure 3 est une vue en élévation de la micro-soufflante de la Figure 2a, avec roue à ailettes visible par transparence,
- la Figure 4 schématise la volute inférieure et le moteur de la micro-soufflante de la Figure 3, avant leur solidarisation,
- les Figures 5 et 6 sont des vues détaillées de la volute inférieure de la micro-soufflante de la Figure 4,
- les Figures 7a et 7b sont des vues de dessus de la micro-soufflante des Figures 3 et 4, sans la roue à ailette, détaillant le couplage de la volute inférieure au capotage,
- la Figure 8 est une vue latérale en coupe de la micro-soufflante des Figures 3 et 4,
- la Figure 9 est une vue grossie de l'intérieur du compartiment à roue à ailettes, après assemblage des volutes,
- la Figure 10 illustre la mise en place du pavillon sur l'âme acoustique équipant la micro-soufflante de la Figure 1,
- la Figure 11 est une vue latérale d'un mode de réalisation du pavillon de la Figure 10, et
- la Figure 12 est une vue latérale d'un mode de réalisation de l'âme acoustique de la Figure 10.

Les Figures ci-annexées schématisent un mode de réalisation d'une micro-soufflante 1, encore appelée compresseur, pour ventilateur médical selon la présente invention.

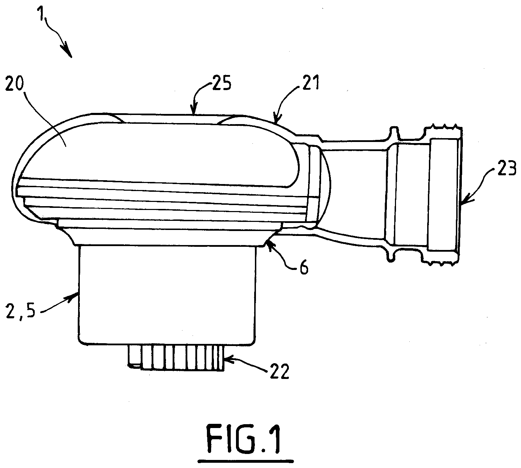

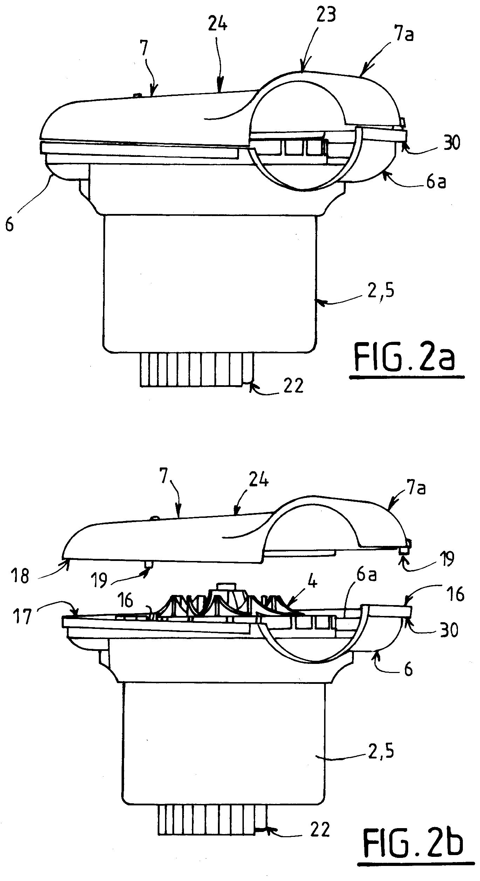

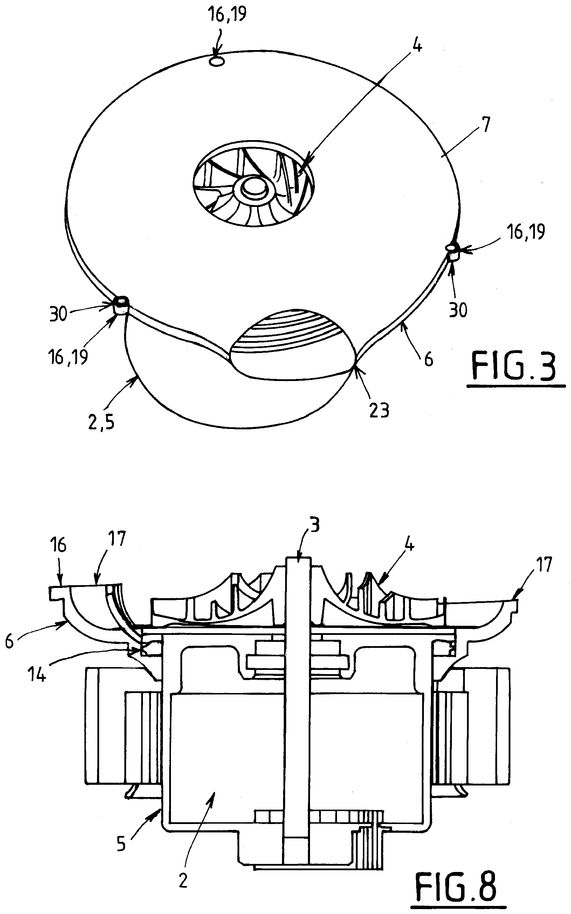

Comme illustré en Figure 1, la micro-soufflante 1 comprend un moteur électrique 2 à arbre rotatif 3 portant une roue à ailettes 4 (voir Figures 2a, 2b et 3) qui permet d'aspirer de l'air et de générer un débit d'air et une pression d'air donnés lorsqu'elle est mise en rotation par le moteur électrique 2, via l'arbre rotatif 3.

L'air aspiré par la roue à ailettes est ensuite expulsé par un conduit de sortie d'air 23 qui permet d'acheminer l'air délivré par la micro-soufflante vers le patient, via une ou plusieurs canalisations de gaz débouchant au niveau d'une interface patient, tel un masque respiratoire ou similaire.

Le moteur électrique 2 est en fait compris dans un capotage 5 ou carter périphérique externe qui est ici de forme cylindrique. Il est alimenté en courant électrique par un faisceau de fils électriques 22.

La micro-soufflante 1 comprend en outre une âme acoustique 20 servant à atténuer les émissions sonores générées notamment par les rotations de la roue à ailettes 4 et le moteur 2, et un pavillon 21 comprenant une ouverture centrale 25 par laquelle l'air peut pénétrer dans la micro-soufflante 1 lorsqu'il est aspiré par la roue à ailettes 4.

Comme illustré en Figures 2a, 2b et 3, la roue à ailettes 4 est agencée entre une volute inférieure 6 et une volute supérieure 7 qui forment l'une et l'autre des demi-coques venant prendre « en sandwich » la roue à ailettes 4. En d'autres termes, les volutes inférieure 6 et supérieure 7, lorsqu'elles sont assemblées l'une à l'autre, définissent entre elles un compartiment interne ou volume creux au sein duquel est agencée la roue à ailettes 4.

La volute inférieure 6 et la volute supérieure 7 sont préférentiellement réalisées en matière plastique mais peuvent être aussi réalisées dans d'autres matériaux, comme en aluminium ou en alliage d'aluminium.

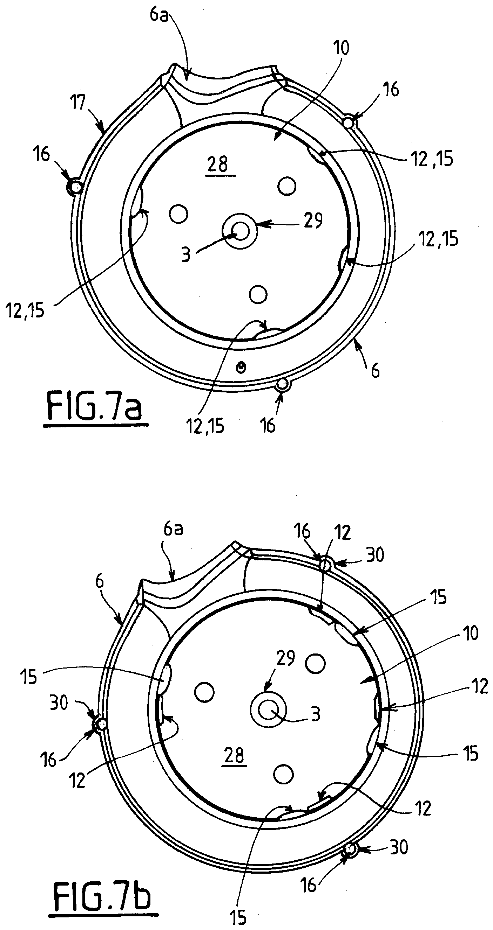

Comme on le comprend au vu des Figures 2a/2b et 7a/7b et , une première partie 6a de la paroi de la volute inférieure 6 et une seconde partie 7a de la paroi de la volute supérieure 7 sont conformées de manière à former une partie, notamment l'entrée, du conduit de sortie d'air 23, lorsque les volutes 6, 7 sont assemblées l'une à l'autre et que lesdites première et seconde parties 6a, 7a de paroi des volutes 6, 7 sont mises en regard l'une de l'autre. En fait, lesdites première et seconde parties 6a, 7a de paroi des volutes 6, 7 forment chacune des portions complémentaires de tube, à savoir des demi-tubes, qui lorsqu'elles sont réunies constituent au moins l'entrée du conduit 23. La longueur de ces portions complémentaires de tube formant les première et seconde parties 6a, 7a est typiquement comprise entre 0,5 cm et 7 cm, de préférence inférieure à 4 cm, typiquement entre 1 et 2,5 cm, par exemple de l'ordre de 1,5 cm.

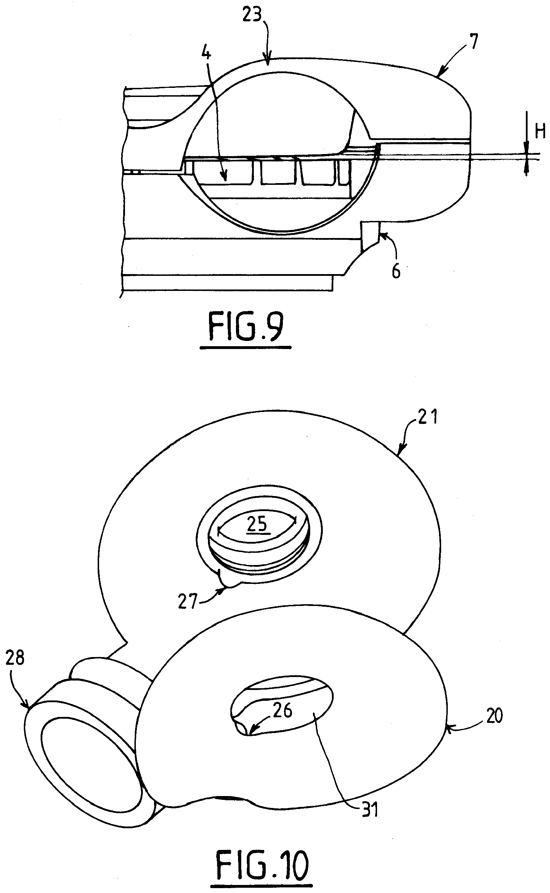

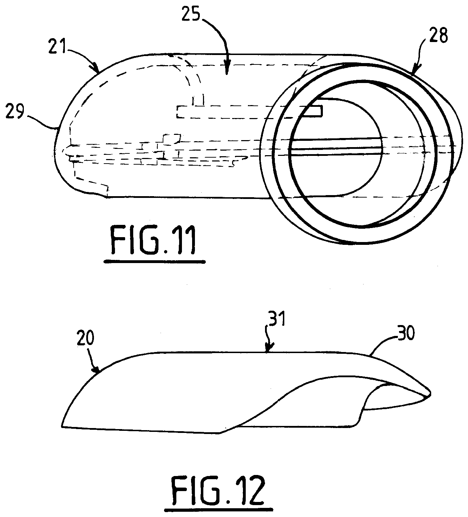

Comme on le voit sur les Figures 10 et 11, la portion tubulaire 28 du pavillon 21 vient se positionner autour des première et seconde parties 6a, 7a de paroi des volutes 6, 7, c'est-à-dire former un manchon autour desdits première et seconde parties 6a, 7a lorsqu'elles sont réunies l'une à l'autre.

Par ailleurs, la volute inférieure 6 comprend un passage central 8 qui vient se positionner autour, c'est-à-dire ceinturer la surface périphérique 9 du capotage 5 du moteur 2. Le diamètre interne dudit passage central 8 est donc supérieur ou égal au diamètre externe du capotage 5, de préférence ils sont approximativement égaux de manière à ce que le contour du passage central 8 vienne épouser le contour externe du capotage 5 du moteur 2.

De même, la volute supérieure 7 comprend également un passage central 24 en regard duquel vient se positionner l'ouverture centrale 25 du pavillon 21 de manière à permettre à l'air aspiré par la roue 4 à ailettes de pénétrer dans le compartiment délimité par les volutes inférieure 6 et supérieure 7 et qui renferme la roue à ailettes 4.

Les volutes inférieure 6 et supérieure 7 ont donc préférentiellement ici chacune une forme générale de coupelle et comprennent chacune un passage en leur centre. Toutefois, selon un autre mode de réalisation possible, l'entrée de l'air dans la volute supérieure 7 pourrait également se faire latéralement et dans ce cas, ladite volute supérieure 7 pourrait comprendre un passage d'air agencé latéralement plutôt qu'en son centre. De façon analogue, elles pourraient avoir également une forme en « colimaçon » (i.e. escargot) plutôt que de coupelle.

Selon la présente invention, afin d'améliorer l'étanchéité fluidique entre la volute inférieure 6 et le capotage ou carter 5 externe du moteur 2 de la micro-soufflante 1, le capotage 5 du moteur et la volute inférieure 6 ont été conformés de façon particulière, comme expliqué ci-après, de manière à coopérer efficacement l'un avec l'autre.

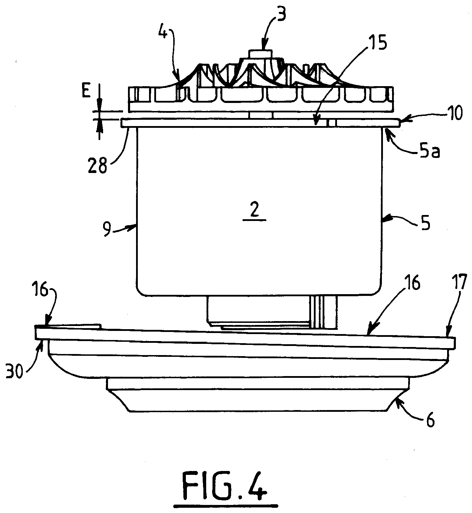

Plus précisément, le capotage 5 du moteur est doté d'un rebord périphérique supérieur 10 formant une couronne autour de la périphérie externe au niveau de la partie supérieure du capotage 5 du moteur 2. Ce rebord périphérique supérieur 10 en forme de couronne se projette radialement en éloignement par rapport à la surface périphérique externe 9 du capotage 5, comme illustré sur la Figure 4.

Selon le mode de réalisation représenté sur les Figures 4 et 7a/7b, le rebord 10 est porté par ou fait partie d'une plaque 28 en forme de disque venant coiffer l'extrémité supérieure 5a du capotage 5, ladite plaque 28 présentant un diamètre supérieur à celui du capotage 5 de manière à constituer ledit rebord périphérique supérieur 10. La plaque 28 est percée en son centre de sorte de former un passage axial 29 pour l'arbre 3 du moteur. Le rebord 10 est donc constitué d'une partie (i.e. bande annulaire) de la région périphérique du disque constituant la plaque 28. La plaque 28 peut être formée d'une seule pièce, par usinage, ou peut être solidairement intégrée aux pièces composant le moteur, par exemple à la paroi périphérique 9 du capotage 5 ou alors fixée à celle-ci par collage, vissage, soudage ou tout autre moyen.

Bien entendu, d'autres modes de réalisation sont envisageables. Ainsi, le rebord 10 peut aussi être formé d'une pièce, par usinage par exemple, avec la paroi périphérique 9 du capotage 5, ou encore être constitué d'une pièce annulaire indépendante qui a été solidarisée à la paroi périphérique 9 du capotage 5, par exemple par collage, vissage, soudage ou tout autre moyen.

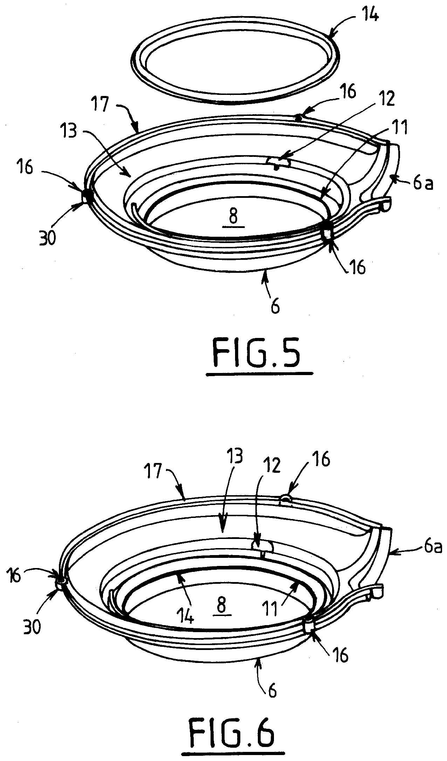

Par ailleurs, comme illustré sur la Figure 5, le passage central 8 de la volute inférieure 6 comprend un épaulement annulaire 11 venant le border intérieurement, c'est-à-dire du côté de la volute inférieure 6 situé en regard de la roue à ailettes 4. Un joint d'étanchéité annulaire 14 est agencé sur ledit épaulement annulaire 11 de manière à s'étendre sur tout le pourtour dudit épaulement 11. L'épaulement 11 et le joint 14 ont ici une forme de couronne circulaire, c'est-à-dire une forme de révolution

Le joint 14 est en matériau flexible, de préférence en élastomère, par exemple en caoutchouc.

De plus, comme montré en Figure 6, la volute inférieure 6 porte plusieurs butées internes 12, à savoir ici trois butées internes 12, qui sont aménagés sur la surface interne 13 de la volute inférieure 6. Les butées internes 12 se projettent radialement en éloignement par rapport à la surface interne 13 de la volute inférieure 6.

Grâce à de tels aménagements, on peut considérablement améliorer l'étanchéité entre la volute inférieure 6 et le capotage 5 du moteur 2 mais aussi simplifier le montage de ces éléments l'un avec l'autre.

En effet, pour ce faire, lors du montage, on vient positionner le rebord périphérique supérieur 10 du capotage 5 du moteur entre le joint d'étanchéité annulaire 14 et les butées internes 12 de la volute inférieure 6. Lesdites butées internes 12 viennent alors appuyer sur le rebord périphérique supérieur 10 du capotage 5 du moteur en le repoussant en direction de l'épaulement annulaire 11 de la volute inférieure 6, ce qui engendre alors une compression par écrasement et déformation du joint d'étanchéité annulaire 14 entre ledit rebord périphérique supérieur 10 du capotage 5 et l'épaulement annulaire 11 de la volute inférieure 6.

En fait, pour permettre cette insertion du rebord périphérique supérieur 10 du capotage 5 entre butées 12 et joint 14, on aménage sur le rebord périphérique supérieur 10 du capotage 5 plusieurs évidements 15, comme visible sur les Figures 7a et 7b notamment, lesquelles évidements 15 sont dimensionnés pour permettre le passage des butées internes 12 de la volute inférieure 6. De préférence, le nombre d'évidements 15 est égal au nombre de butées 12. Avantageusement, on prévoit au moins 3 évidements 15 et au moins 3 butées 12 correspondantes. Ainsi, dans le mode de réalisation présenté sur les Figures, 4 butées 12 et 4 évidements 15 ont été mis en oeuvre, comme visible sur les Figures 7a et 7b.

Une fois que les butées 12 sont complètement mis en regard des évidements 15, puis passées au travers desdits évidements 15, via un déplacement en translation de la volute 6 inférieure par rapport au rebord périphérique supérieur 10 du capotage 5 (illustré en Fig. 7a), il suffit d'opérer un mouvement de rotation de la volute 6 inférieure par rapport au capotage 5 pour venir positionner les butées 12 au contact de la paroi ou couronne annulaire constituant rebord périphérique supérieur 10 du capotage 5, ce qui permet alors aux butées internes 12 d'assurer un maintien solidaire de l'ensemble dans cette position et une compression étanche du joint 14 (illustré en Fig. 7b).

Afin de permettre un tel assemblage, il est indispensable qu'une distance D minimum soit prévue entre le plan comprenant la surface inférieure des butées 12 et le plan incluant la surface supérieure de l'épaulement 11, à savoir une distance d légèrement supérieure à l'épaisseur e du rebord 10, par exemple supérieure de quelques millimètres pour prendre en compte la surépaisseur du joint 14 venant se positionner sur l'épaulement 11, lequel est comprimé lors de l'insertion du rebord 10 entre l'épaulement 11 et les butées 12.

En outre, afin de faciliter la solidarisation de la volute supérieure 7 à la volute inférieure 6, et leur bon alignement l'une par rapport à l'autre, de manière à assurer une bonne étanchéité fluidique entre elles, c'est-à-dire à leur jonction, on prévoit un système de positionnement desdites volutes 6, 7, l'une par rapport à l'autre. Plus précisément, comme visible sur les Figures 2b, 3, 7a, 7b et 8, ce système de positionnement desdites volutes 6, 7 comprend des picots 19 coopérant avec des logements 16 correspondants, au sein desquels ils viennent s'insérer lorsque les volutes 6, 7 sont assemblées l'une à l'autre.

Pour ce faire, on aménage plusieurs logements 16, par exemple trois logements 16, sur la bordure périphérique supérieure 17 de la volute inférieure 6 et, par ailleurs, plusieurs picots 19, par exemple trois picots 19, sur le bord périphérique inférieur 18 de la volute supérieure 7. Chaque picot 19 vient se loger dans l'un des logements 16 lorsque les volutes inférieure 6 et supérieure 7 sont mises au contact l'une de l'autre, c'est-à-dire assemblées l'une à l'autre.

Les logements 16 peuvent, comme ici, être aménagés dans des petites excroissances 30 de la bordure périphérique supérieure 17 de la volute inférieure 6, qui font saillie vers l'extérieur de la volute 6. Toutefois, selon d'autres modes de réalisation, ils peuvent aussi être aménagés directement dans l'arête de ladite bordure périphérique supérieure 17 ou du côté intérieur de la volute 6.

De façon analogue, les picots 19 portés par le bord périphérique inférieur 18 de la volute supérieure 7 peuvent être, comme ici, légèrement décalés extérieurement. Toutefois, selon d'autres modes de réalisation, ils peuvent aussi être portés par l'arête dudit bord périphérique inférieur 18 ou encore légèrement décalés intérieurement.

Les picots 19 sont préférentiellement formés d'une pièce avec le bord périphérique inférieur 18 de la volute supérieure 7, par exemple par moulage. De même, les petites excroissances 30 de la bordure périphérique supérieure 17 de la volute inférieure 6 sont préférentiellement formées d'une pièce avec ladite bordure périphérique supérieure 17.

Quel que soit le mode de réalisation choisi, les logements 16 et les picots 19 doivent être agencés de manière à se faire face et à coopérer ensemble pour que les picots 19 viennent s'insérer facilement dans les logements 16 lors de l'assemblage des volutes 6, 7.

Dans tous les cas, ces paires de picot 19/logement 16 forment donc des points d'arrimage qui permettent un gain de temps lors de l'assemblage et une répétabilité, lors du montage. En d'autres termes, ceci permet non seulement de faciliter le montage de la volute supérieure 7 sur la volute inférieure 6 mais aussi de garantir un bon positionnement de l'une par rapport à l'autre, sans risque de mauvais alignement, donc de garantir une bonne étanchéité fluidique entre les volutes 6, 7. Celles-ci peuvent être éventuellement collées l'une à l'autre. Le montage de la micro-soufflante 1 s'en trouve donc accéléré et l'étanchéité fluidique simplifiée et améliorée.

A noter qu'un mode de réalisation inverse est également envisageable, c'est-à-dire avec les logements 16 agencés sur le bord périphérique inférieur 18 de la volute supérieure 7 et les picots 19 agencés sur la bordure périphérique supérieure 17 de la volute inférieure 6.

Les Figures 10 à 12 schématisent des modes de réalisation possibles du pavillon 21 et de l'âme acoustique 20 d'une micro-soufflante 1 selon la présente invention. Dans tous les cas, le pavillon 21 surmonte l'âme acoustique 20, et cet ensemble surmonte la volute supérieure 7.

On voit que le pavillon 21 a ici une forme générale de coupelle 29 traversée en son centre par l'ouverture centrale 25 et portant latéralement une portion tubulaire 28 en communication fluidique avec l'intérieur du pavillon 21.

Par ailleurs, l'âme acoustique 20 a ici une forme générale annulaire 30 légèrement bombée pour venir épouser le profil externe de la volute supérieure 7, et comporte en son centre un évidement central 31 venant se positionner en regard de l'ouverture centrale 25 du pavillon 21 et du passage central 24 de la volute supérieure 7 de manière à assurer une continuité fluidique entre eux.

Lorsque l'âme acoustique 20 est positionnée sur la volute supérieure 7, elle vient épouser son contour et de la même façon, lorsque le pavillon 21 est agencé sur l'âme acoustique 20, il en épouse également les contours de manière à assurer une bonne solidarisation de l'ensemble.

On réalise préférentiellement le pavillon 21 en un matériau souple, par exemple en silicone, de manière à pouvoir l'enfiler et le positionnement aisément sur l'âme acoustique 20 et la volute supérieure 7.

Afin de faciliter le couplage/alignement correct du pavillon 21 sur l'âme 20, on prévoit un système de détrompage 26, 27, illustré en Figure 10, comprenant un (ou plusieurs) élément en saillie 27, porté ici par le pavillon 21, venant se loger dans un (ou plusieurs) logement associé 26 ayant une forme préférentiellement complémentaire, lequel est porté ici par l'âme acoustique 20, de manière à assurer entre eux un couplage de type mâle/femelle. Bien entendu, de façon alternative, l'élément en saillie 27 pourrait être porté par l'âme acoustique 20 et le logement associé 26 par le pavillon 21, ou plusieurs éléments en saillie 27 et plusieurs logements associés 26 pourraient aussi être agencés à la fois sur le pavillon 21 et sur l'âme acoustique 20 de manière à constituer plusieurs points/zones de couplage/ détrompage.

Plus généralement, le pavillon 21 est préférentiellement réalisé en matériau souple, par exemple en silicone. Par ailleurs, l'âme acoustique 20 est préférentiellement réalisée en matériau élastomère présentant de bonnes propriétés d'amortissement vibratoire.

Avantageusement, le montage de la micro-soufflante 1 de l'invention se fait selon un procédé d'assemblage comprenant les étapes successives suivantes :
a) après avoir positionné la roue à ailettes 4 sur l'arbre rotatif 3 du moteur 2, on agence la volute inférieure 6 munie du joint d'étanchéité 14 autour du capotage 5 du moteur 2 en insérant le capotage 5 du moteur 2 dans le passage central 8 de la volute inférieure 6, comme schématisé en Figure 4. Le positionnement correct dela roue à ailettes 4 sur l'arbre ou axe 3, peut se faire au moyen d'un outillage adapté qui mesure précisément la position de la roue 4 en hauteur par rapport à la partie supérieure de la surface 28, lors de son emmanchement sur l'arbre 3, par exemple un capteur de position ou un détecteur sans contact.
b) on approche ensuite la volute inférieure 6 du rebord périphérique supérieur 10 du capotage 5 en faisant glisser le capotage 5 du moteur 2 dans le passage central 8 de la volute inférieure 6 et on prend soin de positionner correctement, c'est-à-dire d'aligner, les butées 12 portées par la volute inférieure 6 en vis-à-vis des évidements 15 portés par le rebord périphérique supérieur 10 du capotage 5. Cette étape b) est illustrée en Figure 7a qui montre les butées 12 et les évidements 15 positionnés face à face, c'est-à-dire avant le mouvement de translation de l'étape c).
c) puis, on opère un mouvement de translation de la volute inférieure 6 en direction du rebord périphérique supérieur 10 du capotage 5 pour faire passer les butées 12 au travers desdits évidements 15 correspondants.
d) une fois que les butées 12 ont complètement traversé les évidements 15, on opère un mouvement de rotation de la volute inférieure 6 par rapport au capotage 5 du moteur 2 de manière à décaler angulairement les butées 12 par rapport aux évidements 15 et à positionner chaque butée 12 en regard d'une partie du rebord périphérique supérieur 10 du capotage 5 de manière à ce que le rebord périphérique supérieur 10 soit pris en sandwich entre lesdites butées 12 et le joint d'étanchéité 14 porté par l'épaulement annulaire 11 de la volute inférieure 6. Cette étape c) est illustrée en Figure 7b qui montre les butées 12 et les évidements 15 en position angulairement décalée, c'est-à-dire dans une position de blocage solidaire de la volute inférieure 6 et du capotage 5. On assure ainsi un maintien solidaire et fluidiquement étanche du capotage 5 du moteur 2 à la volute inférieure 6 grâce à un écrasement du joint 14, comme illustré en Figure 8. Il se produit en fait une sorte de clipsage par rotation permettant de maintenir la volute inférieure 6 sur le capotage 5 du moteur 2 de manière solidaire et étanche du fait de l'écrasement du joint d'étanchéité 14.

De façon générale, comme schématisé en Figure 4, on veille à ce que la surface inférieure de la roue 4 soit légèrement espacées axialement de la surface supérieure du capotage 5 du moteur, par exemple on prévoit un espacement minimum E entre eux de l'ordre de un à quelques millimètres.

Avantageusement, après positionnement correct de la roue à ailettes 4 sur l'arbre rotatif 3 à l'étape a), on procède à son équilibrage de manière à éviter les vibrations, le bruit généré par celles-ci mais aussi augmenter sa durée de vie. Ceci peut se faire sur une équilibreuse en enlevant de la matière de sorte à diminuer ou supprimer le balourd de la partie tournante à l'aide d'une fraise et d'un système mesurant la position angulaire et la masse du balourd.

En fait, le joint 14 qui est en matériau élastique, va, lorsqu'il est comprimé, aller plaquer, grâce à un effort de rappel dirigé selon l'axe du moteur, les zone d'appuies 12 de la volute inférieure 6 contre la partie supérieure du moteur 2 sur le rebord périphérique supérieur 10. Sur le dessous du disque 10 au niveau de la surface 28 et au niveau de l'épaulement 11, le joint 14a une forme permettant d'assurer une étanchéité fluidique. Le joint 14 assure donc une triple action : étanchéité, plaquage et maintien en position.

Une fois ces éléments mis en place, le montage de la micro-soufflante 1 de l'invention peut se poursuivre selon les étapes suivantes :
e) on assemble les volutes inférieure 6 et supérieure 7, l'une à l'autre, en insérant les picots 19 portés par le bord périphérique inférieur 18 au sein des logements 16 correspondants portés par la volute inférieure 6. Une telle conception à au moins 3 points d'appui situés très précisément par rapport au plan supérieur du moteur est avantageuse car elle permet d'éviter tout problème de mauvais alignement des volutes et la roue 4 se trouve aussi positionnée très précisément par rapport à la surface d'appui permettant ainsi de garantir une hauteur minimum H inférieure à 1 mm, typiquement de l'ordre de 0.1 à 0.4 mm, entre la surface supérieure de la roue 4 et une surface ayant une forme complémentaire à celle du sommet des pales, comme illustré en Figure 9.
f) et enfin, on fixe l'âme acoustique 20 et le pavillon 21 à la volute supérieure 7. L'âme acoustique 20 sert à atténuer les vibrations et le bruit généré par la micro-soufflante 1. Le pavillon 21 sert à maintenir l'âme acoustique 20 sur la volute supérieure 7, à maintenir les deux volutes assemblées entre elles si celles-ci ne sont pas collées, à guider l'air aspiré par la roue à ailettes 4 et à améliorer encore l'étanchéité de l'ensemble.

La micro-soufflante 1 est alors totalement assemblée et prête à l'emploi, et peut dès lors être incorporée à un appareil médical de ventilation assistée, par exemple dans un ventilateur de type MONNAL commercialisée par la Demanderesse.

De façon générale, une micro-soufflante 1 selon l'invention a avantageusement un poids de moins de 500 g et un encombrement limité, à savoir une hauteur entre le bas du capotage 5 du moteur 2 et le dessus du pavillon 21 typiquement inférieure à 15 cm, voire inférieure à 10 cm.

## Revendications

1. Micro-soufflante (1) comprenant un moteur électrique (2) à arbre rotatif (3) portant une roue à ailettes (4), ledit moteur électrique (2) étant compris dans un capotage (5) périphérique, ladite roue à ailettes (4) étant agencée entre une volute inférieure (6) et une volute supérieure (7), la volute inférieure (6) comprenant un passage central (8) venant ceinturer la surface périphérique (9) du capotage (5), le capotage (5) du moteur comprenant un rebord périphérique supérieur (10) se projetant radialement en éloignement par rapport à la surface périphérique externe (9) du capotage (5),
le passage central (8) de la volute inférieure (6) étant bordé intérieurement par un épaulement annulaire (11), la micro-soufflante étant **caractérisée en ce que**:
- un joint d'étanchéité annulaire (14) est agencé sur ledit épaulement annulaire (11),
- la volute inférieure (6) comprend en outre plusieurs butées internes (12) portées par la surface interne (13) de la volute inférieure (6),
- le rebord périphérique supérieur (10) du capotage (5) du moteur est positionné entre le joint d'étanchéité annulaire (14) et les butées internes (12) de la volute inférieure (6), lesdites butées internes (12) venant appuyer sur ledit rebord périphérique supérieur (10) du capotage (5) du moteur de manière à obtenir une compression du joint d'étanchéité annulaire (14) entre ledit rebord périphérique supérieur (10) du capotage (5) et l'épaulement annulaire (11) de la volute inférieure (6), et
- le rebord périphérique supérieur (10) du capotage (5) du moteur (2) comprend plusieurs évidements (15) dimensionnés pour permettre le passage des butées internes (12) de la volute inférieure (6).

2. Micro-soufflante selon la revendication précédente, **caractérisée en ce que** les butées internes (12) de la volute inférieure (6) se projettent radialement en éloignement par rapport à la surface interne (13) de la volute inférieure (6).

3. Micro-soufflante selon l'une des revendications précédentes, **caractérisée en ce que** la volute inférieure (6) comprend au moins 3 butées internes (12).

4. Micro-soufflante selon l'une des revendications précédentes, **caractérisée en ce que** la volute inférieure (6) comprend en outre une bordure périphérique supérieure (17) comprenant plusieurs logements (16), le bord périphérique inférieur (18) de la volute supérieure (7) comprenant plusieurs picots (19), chaque picot (19) venant se loger dans l'un des logements (16) lorsque les volutes inférieure (6) et supérieure (7) sont assemblées l'une à l'autre.

5. Micro-soufflante selon l'une des revendications précédentes, **caractérisée en ce que** la bordure périphérique supérieure (17) de la volute inférieure (6) comprend au moins 3 logements (16) et le bord périphérique inférieur (18) de la volute supérieure (7) comprend au moins 3 picots (19).

6. Micro-soufflante selon la revendication 5, **caractérisée en ce que** le nombre de logements (16) est égal au nombre de picots (19).

7. Micro-soufflante selon l'une des revendications précédentes, **caractérisée en ce que** la volute supérieure (7) est surmontée d'une âme acoustique (20) et/ou d'un pavillon (21).

8. Appareil d'assistance respiratoire comprenant une micro-soufflante selon l'une des revendications précédentes.

9. Procédé d'assemblage d'une micro-soufflante (1) selon l'une des revendications 1 à 7, **caractérisé en ce que** l'on procède selon les étapes successives suivantes :
a) on agence la volute inférieure (6) munie du joint d'étanchéité (14) autour du capotage (5) du moteur (2) en insérant le capotage (5) du moteur (2) dans le passage central (8) de la volute inférieure (6),
b) on positionne chaque butée (12) portée par la volute inférieure (6) en vis-à-vis d'un évidement (15) correspondant porté par le rebord périphérique supérieur (10) du capotage (5),
c) on opère un mouvement de translation de la volute inférieure (6) en direction du rebord périphérique supérieur (10) du capotage (5) pour faire passer les butées (12) au travers desdits évidements (15) correspondants, et
d) après qu'à l'étape c), les butées aient complètement traversé les évidements (15), on opère un mouvement de rotation de la volute inférieure (6) par rapport au capotage (5) du moteur (2) de manière à décaler angulairement les butées (12) par rapport aux évidements (15) et à positionner chaque butée (12) en regard d'une partie du rebord périphérique supérieur (10) du capotage (5) de manière à ce que le rebord périphérique supérieur (10) du capotage (5) soit pris en sandwich entre lesdites butées (12) et le joint d'étanchéité (14) porté par l'épaulement annulaire (11) de la volute inférieure (6), en assurant ainsi un maintien solidaire et fluidiquement étanche du capotage (5) du moteur (2) à la volute inférieure (6).

10. Procédé selon la revendication 9, **caractérisé en ce que**, préalablement à l'étape a), on positionne la roue à ailettes (4) sur l'arbre rotatif (3) du moteur (2).

11. Procédé selon l'une des revendications 9 ou 10, **caractérisé en ce qu'**après l'étape d), il comprend l'étape e) suivante :
e) on assemble les volutes inférieure (6) et supérieure (7), l'une à l'autre, en insérant les picots (19) portés par le bord périphérique inférieur (18) de la volute supérieure (7) au sein des logements (16) correspondants portés par la bordure périphérique supérieure (17) de la volute inférieure (6).

12. Procédé selon l'une des revendications 9 à 11, **caractérisé en ce qu'**après l'étape e), il comprend l'étape f) suivante :
f) on fixe une âme acoustique (20) et/ou un pavillon (21) à la volute supérieure (7).

13. Procédé selon l'une des revendications 9 à 12, **caractérisé en ce qu'**après le positionnement la roue à ailettes (4) sur l'arbre rotatif (3) du moteur (2), on opère un équilibrage de ladite roue à ailettes (4) sur ledit arbre rotatif (3).

## Patentansprüche

1. Mikrogebläse (1) mit einem Elektromotor (2) mit drehbarer Welle (3), die ein Flügelrad (4) trägt, wobei der Elektromotor (2) in einer umfänglichen Verkleidung (5) enthalten ist, wobei das Flügelrad (4) zwischen einem unteren Spiralgehäuse (6) und einem oberen (7) Spiralgehäuse angeordnet ist, wobei das untere Spiralgehäuse (6) einen zentralen Durchlass (8) umfasst, der die Umfangsoberfläche (9) der Verkleidung (5) umschließt, wobei die Verkleidung (5) des Motors einen obere Umfangsrand (10) umfasst, der radial von der äußeren Umfangsoberfläche (9) der Verkleidung (5) fortweisend hervorsteht,
wobei der zentrale Durchlass (8) des unteren Spiralgehäuses (6) innen von einer ringförmigen Schulter (11) begrenzt wird, wobei das Mikrogebläse **dadurch gekennzeichnet ist, dass**:
- eine ringförmige Dichtung (14) an der ringförmigen Schulter (11) angeordnet ist,
- das untere Spiralgehäuse (6) weiter mehrere innere Anschläge (12) umfasst, die von der Innenseite (13) des unteren Spiralgehäuses (6) getragen werden,
- der obere Umfangsrand (10) der Verkleidung (5) des Motors zwischen der ringförmigen Dichtung (14) und den inneren Anschlägen (12) des unteren Spiralgehäuses (6) angeordnet ist, wobei die inneren Anschläge (12) gegen den oberen Umfangsrand (10) der Verkleidung (5) des Motors gedrückt werden, so dass zwischen dem oberen Umfangsrand (10) der Verkleidung (5) und der ringförmigen Schulter (11) des unteren Spiralgehäuses (6) ein Zusammendrücken der ringförmigen Dichtung (14) erzielt wird und
- der obere Umfangsrand (10) der Verkleidung (5) des Motors (2) mehrere Aussparungen (15) aufweist, die so bemessen sind, dass sie den Durchlass der inneren Anschläge (12) des unteren Spiralgehäuses (6) ermöglichen.

2. Mikrogebläse nach dem vorstehenden Anspruch, **dadurch gekennzeichnet, dass** die inneren Anschläge (12) des unteren Spiralgehäuses (6) radial von der Innenseite (13) des unteren Spiralgehäuses (6) fortweisend hervorstehen.

3. Mikrogebläse nach einem der vorstehenden Ansprüche, **dadurch gekennzeichnet, dass** das untere Spiralgehäuse (6) mindestens drei innere Anschläge (12) umfasst.

4. Mikrogebläse nach einem der vorstehenden Ansprüche, **dadurch gekennzeichnet, dass** das untere Spiralgehäuse (6) weiter eine obere Umfangsumrandung (17) mit mehreren Aufnahmen (16) umfasst, wobei die untere Umfangskante (18) des oberen Spiralgehäuses (7) mehrere Stifte (19) umfasst, wobei jeder Stift (19) von einer der Aufnahmen (16) aufgenommen wird, wenn das untere (6) und das obere (7) Spiralgehäuse miteinander verbunden sind.

5. Mikrogebläse nach einem der vorstehenden Ansprüche, **dadurch gekennzeichnet, dass** die obere Umfangsumrandung (17) des unteren Spiralgehäuses (6) mindestens drei Aufnahmen (16) umfasst und die untere Umfangskante (18) des oberen Spiralgehäuses (7) mindestens drei Stifte (19) umfasst.

6. Mikrogebläse nach Anspruch 5, **dadurch gekennzeichnet, dass** die Anzahl der Aufnahmen (16) und die Anzahl der Stifte (19) übereinstimmen.

7. Mikrogebläse nach einem der vorstehenden Ansprüche, **dadurch gekennzeichnet, dass** oberhalb des oberen Spiralgehäuses (7) ein Akustikkern (20) und/oder ein Schalltrichter (21) angebracht sind.

8. Vorrichtung zur Atmungsunterstützung mit einem Mikrogebläse nach einem der vorhergehenden Ansprüche.

9. Verfahren zum Zusammenbau eines Mikrogebläses (1) nach einem der Ansprüche 1 bis 7, **dadurch gekennzeichnet, dass** das Verfahren nacheinander die folgenden Schritte umfasst:
a) das mit der Dichtung (14) versehene untere Spiralgehäuse (6) wird um die Verkleidung (5) des Motors (2) herum angeordnet, indem die Verkleidung (5) des Motors (2) in den zentralen Durchlass (8) des unteren Spiralgehäuses (6) eingeführt wird,
b) jeder Anschlag (12), der von dem unteren Spiralgehäuse (6) getragen wird, wird gegenüber von einer entsprechenden Aussparung (15) positioniert, die vom oberen Umfangsrand (10) der Verkleidung (5) getragen wird,
c) durch Ausüben einer Translationsbewegung des unteren Spiralgehäuses (6) in Richtung auf den oberen Umfangsrand (10) der Verkleidung (5) werden die Anschläge (12) durch die entsprechenden Aussparungen (15) geführt, und
d) nachdem in Schritt c) die Anschläge komplett durch die Aussparungen (15) geführt wurden, wird eine Rotationsbewegung des unteren Spiralgehäuses (6) bezogen auf die Verkleidung (5) des Motors (2) ausgeübt, so dass die Anschläge (12) bezogen auf die Aussparungen (15) winkelförmig versetzt sind und jeder Anschlag (12) gegenüber von einem Teil des oberen Umfangsrands (10) der Verkleidung (5) positioniert ist, so dass der obere Umfangsrand (10) der Verkleidung (5) zwischen den Anschlägen (12) und der Dichtung (14), die von der ringförmigen Schulter (11) des unteren Spiralgehäuses (6) getragen wird, eingeklemmt ist und auf diese Weise ein fester und flüssigkeitsdichter Halt der Verkleidung (5) des Motors (2) am unteren Spiralgehäuse (6) gewährleistet ist.

10. Verfahren nach Anspruch 9, **dadurch gekennzeichnet, dass** vor Ausführung von Schritt a) das Flügelrad (4) auf die drehbare Welle (3) des Motors (2) positioniert wird.

11. Verfahren nach Anspruch 9 oder 10, **dadurch gekennzeichnet, dass** das Verfahren nach Ausführung von Schritt d) den folgenden Schritt e) umfasst:
e) das obere (6) und untere (7) Spiralgehäuse werden miteinander verbunden, indem die Stifte (19), die von der unteren Umfangskante (18) des oberen Spiralgehäuses (7) getragen werden, in die entsprechenden Aufnahmen (16), die von der oberen Umfangsumrandung (17) des unteren Spiralgehäuses (6) getragen werden, eingeführt werden.

12. Verfahren nach einem der Ansprüche 9 bis 11, **dadurch gekennzeichnet, dass** das Verfahren nach Ausführung von Schritt e) den folgenden Schritt f) umfasst:
f) am oberen Spiralgehäuse (7) werden ein Akustikkern (20) und/oder ein Schalltrichter (21) befestigt.

13. Verfahren nach einem der Ansprüche 9 bis 12, **dadurch gekennzeichnet, dass** nach dem Positionieren des Flügelrads (4) auf der drehbaren Welle (3) des Motors (2) das Flügelrad (4) auf der drehbaren Welle (3) gewuchtet wird.

## Claims

1. Micro-blower (1) comprising an electric motor (2) having a rotary shaft (3) carrying a vane wheel (4), said electric motor (2) being contained in a peripheral cowling (5), said vane wheel (4) being arranged between a lower volute (6) and an upper volute (7), the lower volute (6) having a central passage (8) encircling the peripheral surface (9) of the cowling (5), the cowling (5) of the motor having an upper peripheral lip (10) projecting radially away from the outside peripheral surface (9) of the cowling (5),
the central passage (8) of the lower volute (6) being bordered inwardly by an annular shoulder (11), the micro-blower being **characterized in that**:
- an annular seal (14) is arranged on said annular shoulder (11);
- the lower volute (6) also comprises a plurality of internal abutments (12) carried by the inside surface (13) of the lower volute (6),
- the upper peripheral lip (10) of the cowling (5) of the motor is positioned between the annular seal (14) and the internal abutments (12) of the lower volute (6), said internal abutments (12) coming to bear against said upper peripheral lip (10) of the cowling (5) of the motor in such a manner as to obtain compression of the annular seal (14) between said upper peripheral lip (10) of the cowling (5) and the annular shoulder (11) of the lower volute (6), and
- the upper peripheral lip (10) of the cowling (5) of the motor (2) comprises a plurality of recesses (15) dimensioned to enable the internal abutments (12) of lower volute (6) to pass through.

2. Micro-blower according to the preceding claim, **characterized in that** the internal abutments (12) of the lower volute (6) project radially away from the inside surface (13) of the lower volute (6).

3. Micro-blower according to either of the preceding claims, **characterized in that** the lower volute (6) comprises at least three internal abutments (12).

4. Micro-blower according to any preceding claim, **characterized in that** the lower volute (6) also comprises an upper peripheral rim (17) comprising plurality of peg holes (16), the lower peripheral edge (18) of the upper volute (7) comprising a plurality of pegs (19), each peg (19) coming to be received in one of the peg holes (16) when the lower volute (6) and the upper volute (7) are assembled together.

5. Micro-blower according to any preceding claim, **characterized in that** the upper peripheral rim (17) of the lower volute (6) comprises at least three peg holes (16) and the lower peripheral edge (18) of the upper volute (7) comprises at least 3 pegs (19).

6. Micro-blower according to claim 5, **characterized in that** the number of peg holes (16) is equal to the number of pegs (19).

7. Micro-blower according to any preceding claim, **characterized in that** the upper volute (7) underlies an acoustic core (20) and/or a shroud (21).

8. Breathing assistance apparatus comprising a micro-blower according to any preceding claim.

9. Method of assembling a micro-blower (1) according to any one of claims 1 to 7, **characterized in that** it consists in performing the following successive steps:
a) arranging the lower volute (6) as equipped with the seal (14) around the cowling (5) of the motor (2) by inserting the cowling (5) of the motor (2) into the central passage (8) of the lower volute (6);
b) positioning each abutment (12) carried by the lower volute (6) facing a corresponding recess (15) carried by the upper peripheral lip (10) of the cowling (5);
c) moving the lower volute (6) in translation towards the upper peripheral lip (10) of the cowling (5) so as to cause the abutments (12) to pass through said corresponding recesses (15); and
d) once the abutments have passed fully through the recesses (15) in step c), moving the lower volute (6) in rotation relative to the cowling (5) of the motor (2) in such a manner as to offset the abutments (12) angularly relative to the recesses (15) and to position each abutment (12) facing a portion of the upper peripheral lip (10) of the cowling (5) in such a manner that the upper peripheral lip (10) of the cowling (5) is sandwiched between said abutments (12) and the seal (14) carried by the annular shoulder (11) of the lower volute (6), thereby ensuring that the cowling (5) of the motor (2) is held securely and in fluid-tight manner on the lower volute (6).

10. Method according to claim 9, **characterized in that**, before step a), the vane wheel (4) is positioned on the rotary shaft (3) of the motor (2).

11. Method according to claim 9 or 10, **characterized in that**, after step d), it comprises the following step e):
e) assembling together the lower volute (6) and the upper volute (7) by inserting the pegs (19) carried by the lower peripheral edge (18) of the upper volute (7) into the corresponding peg holes (16) carried by the upper peripheral rim (17) of the lower volute (6).

12. Method according to any one of claims 9 to 11, **characterized in that**, after step e), it comprises the following step f):
f) fastening an acoustic core (20) and/or a shroud (21) to the upper volute (7).

13. Method according to any one of claims 9 to 12, **characterized in that**, after the vane wheel (4) has been positioned on the rotary shaft (3) of the motor (2), the vane wheel (4) is balanced on said rotary shaft (3).
